# EUROPÄISCHE PATENTANMELDUNG

(11) **EP 2 204 137 A1**
(43) Veröffentlichungstag der Anmeldung: **07.07.2010**
(21) Anmeldenummer: 09015705.8
(22) Anmeldetag: 18.12.2009
(51) Int. Cl.: A61C 8/00

(54) **Implantat**

(30) Priorität: 19.12.2008 DE 102008055551
(71) Anmelder: Zolow, Roman, 4704 Niederbipp (CH)
(72) Erfinder: Zolow, Roman, 4704 Niederbipp (CH)
(74) Vertreter: Lemcke, Brommer & Partner

(57) **Zusammenfassung**

Die Erfindung betrifft ein Implantat (1) zum Einbringen in einen Körper, umfassend einen Implantatkörper (1a) sowie zumindest einen chemischen Stoff oder eine Stoffkombination. Der Implantatkörper (1a) ist dabei zur dauerhaften Abgabe des zumindest einen chemischen Stoffes oder der Stoffkombination in den Körper ausgebildet und umfasst Mittel (6) zur dauerhaften Festlegung des Implantats im Körper.

## Beschreibung

Die Erfindung betrifft ein Implantat zum Einbringen in einen Körper, umfassend ein Implantatkörper sowie zumindest einen chemischen Stoff oder eine Stoffkombination.

Derartige Implantate sind im Bereich der Schwangerschaftsverhütung bekannt. Dabei wird ein kleiner dünner und biegsamer Stab aus Kunststoff als Implantatkörper verwendet, welcher ein Gestagen zur Kontrazeption enthält. Der Stab gibt dabei geringste Mengen des Gestagens kontinuierlich in die Blutbahn ab und verhindert auf diese Weise eine Schwangerschaft.

Der Stab wird an der Innenseite eines Oberarms einer Frau unter die Haut eingesetzt. Nach ca. 3 Jahren ist der Gestagenvorrat im Stab verbraucht. Um eine Schwangerschaft auch in der Zukunft zu verhindern, muss der Stab durch einen Schnitt in die Haut des Oberarms entfernt und wieder ein neuer Stab eingesetzt werden.

Nachteilig dabei ist, dass nach Verbrauch des Gestagens der Stab durch einen Eingriff ersetzt werden muss. Ein weiterer Nachteil ist, dass der unter die Haut eingesetzte Kunststoffstab nur für wenige chemische Stoffe oder Medikamente als Depot dienen kann.

Aufgabe der vorliegenden Erfindung ist es daher, die Handhabung eines Implantats zu verbessern, gleichzeitig den Einsatzbereich des Implantats zu erweitern und schließlich eine einfache Einbringung in einen Körper und kostengünstige Herstellung eines solchen Implantats zu ermöglichen.

Die vorliegende Erfindung löst diese Aufgabe bei einem Implantat zum Einbringen in einen Körper, umfassend einen Implantatkörper sowie zumindest einen chemischen Stoff oder eine Stoffkombination dadurch, dass das Implantat zur dauerhaften Abgabe des zumindest einen chemischen Stoffes oder der Stoffkombination in den Körper ausgebildet ist und Mittel zur dauerhaften Festlegung des Implantats im Körper umfasst.

Der Implantatkörper ermöglicht auf diese Weise eine Abgabe des chemischen Stoffs oder der Stoffkombination über lange Zeiträume. Ein chemischer Stoff, beispielsweise in Form eines Medikaments, kann auf diese Weise einfach und zuverlässig in einen Körper gelangen. Gleichzeitig kann das Implantat durch die Mittel zur dauerhaften Festlegung im Körper an einer optimalen Stelle im Körper angeordnet werden: So kann beispielsweise das Implantat mit einem chemischen Stoff zur Bekämpfung eines lokalen Tumors in dessen unmittelbarer Nähe angeordnet werden, um eine möglichst hohe lokale Konzentration des chemischen Stoffes, welcher den Tumor bekämpft, zu erreichen. Auch kann damit insgesamt eine deutlich kleinere Menge des chemischen Stoffs verwendet werden; unangenehme Nebenwirkungen für einen Patienten werden damit deutlich vermindert.

Vorteilhafterweise umfasst der implantatkörper einen Hohlraum zur Aufnahme eines Gegenstandes. Dies erhöht wesentlich die Flexibilität bzw. Einsatzmöglichkeiten des Implantats, da der Gegenstand im Implantatkörper angeordnet werden kann. Ist das Implantat beispielsweise außen nachgiebig ausgestaltet, so kann der Gegenstand im Inneren eine Versteifung des Implantats und damit eine zuverlässigere Festlegung des Implantats im Körper ermöglichen.

Besonders vorteilhaft ist es dabei, wenn der Hohlraum eine Öffnung zum Einbringen des Gegenstandes umfasst. Auch dies erhöht die Flexibilität des Implantats wesentlich: Über die Öffnung kann der Gegenstand ohne weiteres einfach und schnell eingebracht und auch ausgetauscht werden, wenn sich die Voraussetzungen für das ursprüngliche Einsetzen des Implantats in den Körper geändert haben. Wird, wie oben erwähnt, der Gegenstand zur Versteifung des Implantats eingesetzt, so kann später gegebenenfalls eine schwächere oder stärkere Versteifung eingesetzt werden.

Weiter ist es insbesondere dabei vorteilhaft, wenn die Öffnung des Hohlraums mittels einer Schraube verschließbar ist. Die Schraube ermöglicht ein einfaches und schnelles sowie zuverlässiges Verschließen des Hohlraumes und dichtet den Hohlraum gegebenenfalls mittels einer zusätzlichen Dichtung gegenüber der Umgebung ab. Gleichzeitig wird ein in das Implantat eingebrachter Gegenstand zuverlässig im Hohlraum angeordnet bzw. eingeschlossen.

Zweckmäßigerweise umfasst der Gegenstand einen Träger für den chemischen Stoff oder die Stoffkombination, insbesondere für zumindest ein Medikament. Auf diese Weise ist der Einsatz des Implantats für die verschiedensten chemischen Stoffe oder Medikamente möglich, da das Implantat nicht als Träger für den chemischen Stoff direkt dient sondern lediglich mittelbar als Halterung für den Träger für den chemischen Stoff bzw. das Medikament. Der Einsatzbereich eines solchen Implantats wird damit wesentlich erweitert.

Vorteilhafterweise besteht der Träger aus Kunststoff und ist insbesondere in Form einer Kapsel ausgebildet. Ist der Träger aus Kunststoff ausgebildet, lassen sich eine Vielzahl von chemischen Stoffen oder Medikamenten an den Träger binden. Ist der Träger in Form einer Kapsel ausgebildet, so müssen keine Spezialanfertigungen für den Träger zum Einbringen in den Hohlraum angefertigt werden; es kann auf die günstige und leicht zu beschaffende Kapselform für Medikamente zurückgegriffen werden.

Zweckmäßigerweise umfassen die Mittel zum Festlegen des Implantatkörpers im Körper ein Gewinde. Dies erleichtert die Festlegung und den Verbleib des Implantatkörpers an einer optimalen Stelle. Gleichzeitig kann das Implantat nicht durch Bewegungen, etc. des Körpers aus seiner eingesetzten optimalen Position verrutschen.

Um eine zuverlässige und kontinuierliche Abgabe des zumindest einen chemischen Stoffes oder der Stoffkombination in den Körper zu ermöglichen, steht der Hohlraum des Implantats über zumindest eine weitere Öffnung mit dem Körper in Verbindung. Das Implantat verwächst nach gewisser Zeit zumindest teilweise mit dem Körper, wobei Körpermaterial teilweise in die Öffnung eindringt und eine bessere Abgabe des chemischen Stoffes an den Körper ermöglicht.

Zweckmäßigerweise ist der Implantatkörper in einem Knochen des Körpers festlegbar. Neben einer zuverlässigen Festlegung des Implantats durch die Anordnung des Implantatkörpers in einem Knochen, ist damit gleichzeitig auch eine Abgabe des Medikaments nicht nur mittelbar über den Blutkreislauf in den Knochen, sondern auch direkt in diesen möglich.

Vorteilhafterweise ist die weitere Öffnung in Form eines Schlitzes und/oder einer ovalen Pore ausgebildet. Durch diese Ausbildung der weiteren Öffnung wird eine optimale Verbindung zwischen dem Hohlraum des Implantatkörpers und der äußeren Umgebung des Implantats ermöglicht. Ein im Hohlraum des Implantats befindlicher Träger für ein Medikament kann auf diese Weise stetig und zuverlässig über lange Zeiträume das Medikament Ober die Öffnung an den das Implantat umgebenden Körper abgeben.

Um eine noch bessere Integration des Implantats in den Körper zu ermöglichen, ist es zweckmäßig, wenn eine äußere Oberfläche des Implantats hydrophil ausgebildet ist und/oder Mikrorauhigkeiten umfasst. Wird das Implantat beispielsweise in einen Knochen eingebracht, so wächst der umgebende Knochen schneller als gewöhnlich um das Implantat und ermöglicht eine zuverlässige Festlegung des Implantats.

Vorteilhafterweise besteht der Implantatkörper aus Titan. Da Titan sich biologisch neutral verhält und keine allergischen oder Fremdkörperreaktionen auslöst, ermöglicht die Verwendung von Titan als Material für den Implantatkörper eine besonders gute Verträglichkeit für einen Patienten. Wird der Implantatkörper beispielsweise in einem Knochen festgelegt, führen die biologischen Eigenschaften des Knochens dazu, dass dieser eine direkte kristallographische Verbindung mit der an der Oberfläche des Implantatkörpers auftretenden Oxidschicht bildet (Osseointegration).

Vorteilhafterweise ist das Implantat als Zahnimplantat ausgebildet. Das Implantat umfasst damit gleich zwei Funktionen: Zum einen ermöglicht es Zahnersatz, zum anderen ermöglicht das Implantat eine dauerhafte Abgabe des zumindest einen chemischen Stoffes oder der Stoffkombination. Damit kann bei nur einem Arzt beispielsweise sowohl eine Langzeitmedikation mit einem chemischen Stoff oder einem Medikament erfolgen als auch eine Überprüfung oder einen Ersatz von erkrankten Zähnen. Ist zum Beispiel ein Patient an Diabetes dauerhaft erkrankt, kann dieser, wenn er ein Zahnimplantat benötigt, ein erfindungsgemäßes Zahnimplantat einsetzen lassen. Das Implantat stellt dann sowohl die Funktion als Zahnersatz zur Verfügung als auch zur Langzeitmedikation, beispielsweise durch die dauerhafte Abgabe von Insulin zur Diabetesbehandlung.

Weitere Vorteile und Merkmale der Erfindung ergeben sich aus der nachfolgenden Beschreibung eines Ausführungsbeispiels anhand der Zeichnungen. Dabei zeigt
- Figur 1: den schematischen Aufbau eines erfindungsgemäßen Implantats in einer Seitenansicht;
- Figur 2: das Implantat gemäß Figur 1 nach dem Einbringen in einen Knochen.

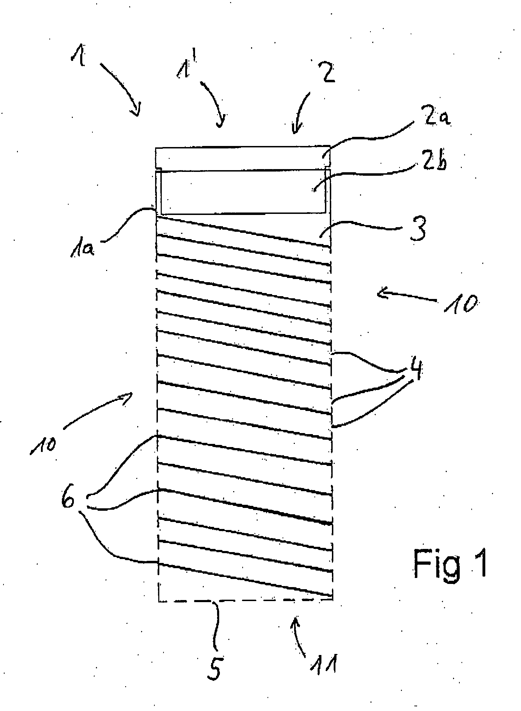

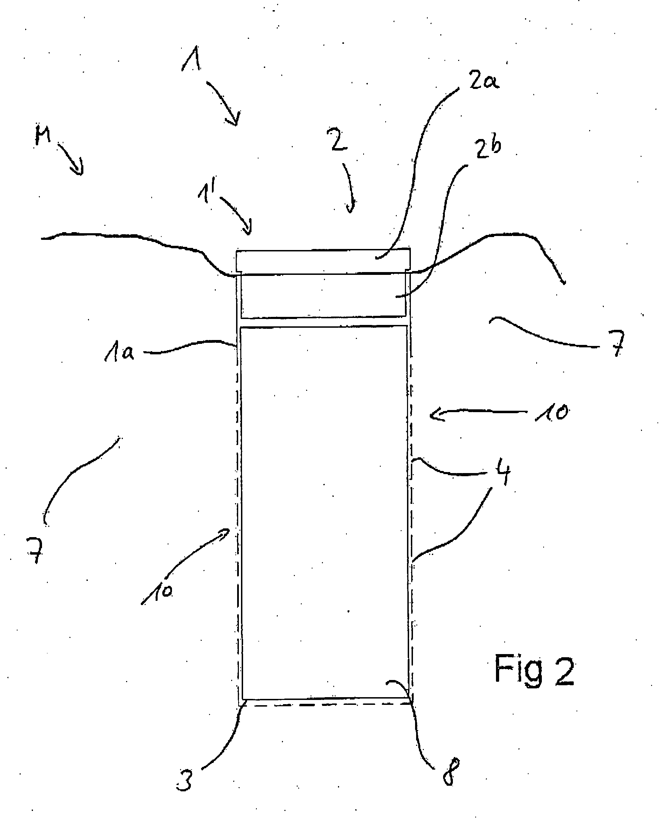

Figur 1 zeigt ein erfindungsgemäßes im Wesentlichen zylindrisches Implantat 1 mit einem im Wesentlichen U-förmigen Implantatkörper 1 a. Im Inneren des Implantatkörpers 1a ist ein Hohlraum 3 zur Aufnahme einer Medikamentenkapsel (siehe Figur 2) angeordnet. Der Hohlraum 3 bzw. der Implantatkörper 1a weist eine obere Öffnung 1' auf, die mit einer Schraube 2, umfassend einen Schraubenkopf 2a und ein Schraubengewinde 2b, verschließbar ist. Das Schraubengewinde 2b greift dabei in ein entsprechendes Gewinde (nicht gezeigt) des Implantatkörpers 1a ein. Weiterhin umfasst der Implantatkörper 1a auf seiner Außenseite an senkrechten Längsseiten 10 ein zumindest teilweise umlaufendes Gewinde 6 auf zum Festlegen des Implantats 1 in einem Körper. An einem waagrechten Boden 11 des Implantats 1, welcher der Öffnung 1' gegenüberliegt, sind Poren bzw. Schlitze 5 ausgebildet, ebenso an den senkrechten Längsseiten 10 des Implantatkörpers 1a (Poren bzw. Öffnungen 4), welche eine Verbindung zwischen dem Hohlraum 3 des Implantates 1 und einer Umgebung des Implantats 1 ermöglichen.

Figur 2 zeigt nun das erfindungsgemäße Implantat 1 festgelegt in einem umgebenden Kieferknochen 7. Die Öffnung 1' des Implantats 1 ist dabei mit der Schraube 2 verschlossen. Im Inneren des Implantats 1 ist in dem Hohlraum 3 eine Medikamentenkapsel 8 angeordnet. Die Medikamentenkapsel 8 ist dabei über an den senkrechten Längsseiten 10 des Implantates 1 angeordnete Öffnungen 4 mit dem umgebenden Knochen 7 mittelbar verbunden. Der Medikamententräger 8 gibt dabei über die Öffnungen 4 das Medikament an den umgebenden Knochen 7 und dann weiter in einen Blutkreislauf ab. Die Schraube 2 dichtet den Hohlraum 3 bzw. die im Hohlraum 3 befindliche Medikamentenkapsel 8 gegenüber einer Mundhöhle M eines Menschen ab.

Wird die Schraube 2 entfernt, kann der Hohlraum 3 des Implantatkörpers 1a eingesehen werden und gegebenenfalls im Hohlraum 3 Veränderungen vorgenommen werden. Hierunter fällt insbesondere ein Austausch einer Medikamentenkapsel 8 oder auch beispielsweise die temporäre Einbringung eines Werkzeugs zur Reinigung der Schlitze und/oder der Poren 4. Der Implantatkörper 1a weist dabei eine zum Knochen 7 zugewandte äußere Oberfläche mit hydrophiler Struktur auf. Weiterhin umfasst diese Mikrorauhigkeiten zur verbesserten Integration in den Kieferknochen 7.

Im Rahmen dieser Erfindung können mittels des Medikamententrägers 8 eine Vielzahl von Medikamenten in den Körper eingebracht werden. Diese umfassen beispielsweise Kontrazeptiva, Insuline, Schmerzmittel, Medikamente zur Tumortherapie, Marker, etc. Weiterhin liegt es im Rahmen dieser Erfindung, dass das Implantat 1 nicht nur die Einbringung von chemischen Stoffen oder Stoffkombinationen in einen Körper über lange, sondern auch über kurze Zeiträume zur Kurzzeitmedikation umfasst

## Patentansprüche

1. Implantat (1) zum Einbringen in einen Körper, umfassend einen Implantatkörper (1a) sowie zumindest einen chemischen Stoff oder eine Stoffkombination, **dadurch gekennzeichnet, dass**
der Implantatkörper (1a) zur dauerhaften Abgabe des zumindest einen chemischen Stoffes oder der Stoffkombination in den Körper ausgebildet ist und Mittel (6) zur dauerhaften Festlegung des Implantats im Körper umfasst.

2. Implantat nach Anspruch 1,
**dadurch gekennzeichnet, dass**
der Implantatkörper (1a) einen Hohlraum (3) zur Aufnahme eines Gegenstandes (8) umfasst.

3. Implantat nach Anspruch 2,
**dadurch gekennzeichnet, dass**
der Hohlraum (3) eine Öffnung (1') zum Einbringen des Gegenstandes (8) umfasst.

4. Implantat nach Anspruch 3,
**dadurch gekennzeichnet, dass**
die Öffnung (1') des Hohlraums (3) mittels einer Schraube (2) verschließbar ist.

5. Implantat nach Anspruch 2,
**dadurch gekennzeichnet,**
**dass** der Gegenstand (8) einen Träger für den chemischen Stoff oder die Stoffkombination, insbesondere für zumindest ein Medikament umfasst.

6. Implantat nach Anspruch 5,
**dadurch gekennzeichnet, dass**
der Träger (8) aus Kunststoff besteht und insbesondere in Form einer Kapsel ausgebildet ist.

7. Implantat nach Anspruch 1,
**dadurch gekennzeichnet, dass**
die Mittel (6) zum Festlegen des Implantatkörpers im Körper ein Gewinde umfassen.

8. Implantat nach Anspruch 1,
**dadurch gekennzeichnet, dass**
der Hohlraum (3) des Implantats (1) über zumindest eine weitere Öffnung (4, 5) mit dem Körper in Verbindung steht.

9. Implantat nach Anspruch 1,
**dadurch gekennzeichnet, dass**
der Implantatkörper (1a) in einem Knochen (7) des Körpers festlegbar ist.

10. Implantat nach Anspruch 8,
**dadurch gekennzeichnet, dass**
die weitere Öffnung (4, 5) in Form eines Schlitzes und/oder einer ovalen Pore ausgebildet ist.

11. Implantat nach zumindest Anspruch 1,
**dadurch gekennzeichnet, dass**
eine äußere Oberfläche des Implantats (1) hydrophil ausgebildet ist und/oder Mikrorauhigkeiten umfasst.

12. Implantat nach zumindest Anspruch 1,
**dadurch gekennzeichnet, dass**
zumindest der Implantatkörper (1a) aus Titan besteht.

13. Implantat nach zumindest Anspruch 1,
**dadurch gekennzeichnet, dass**
das Implantat (1) als Zahnimplantat ausgebildet ist.
